(19) **Europäisches Patentamt / European Patent Office / Office européen des brevets**

(11) **EP 1 895 298 A1**

(12) # EUROPÄISCHE PATENTANMELDUNG

(43) Veröffentlichungstag:
**05.03.2008 Patentblatt 2008/10**

(51) Int Cl.:
*G01N 33/46* *(2006.01)*    *G01N 21/898* *(2006.01)*

(21) Anmeldenummer: **07450147.9**

(22) Anmeldetag: **24.08.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK YU**

(30) Priorität: **31.08.2006  AT 14472006**

(71) Anmelder: **Karl, Buchegger**
**8225 Pöllau (AT)**

(72) Erfinder: **Karl, Buchegger**
**8225 Pöllau (AT)**

(74) Vertreter: **Wirnsberger, Gernot**
**Mühlgasse 3**
**8700 Leoben (AT)**

(54) **Verfahren zur Bestimmung der Drehwüchsigkeit eines Holzstammes**

(57)    Die Erfindung betrifft ein Verfahren zur Bestimmung der Drehwüchsigkeit eines Holzstammes (1). Ein solches Verfahren kann erfindungsgemäß auf einfache Weise durchgeführt werden, wenn der Holzstamm (1) zumindest teilweise entrindet wird und in einem entrindeten Bereich ein allenfalls von einem parallel zur Längsachse (X) des Hoizstammes (1) abweichender Verlauf der Längsriefen (5) des Holzstammes (1) ermittelt und daraus die Drehwüchsigkeit bestimmt wird.

FIG.1

EP 1 895 298 A1

**Beschreibung**

Die Erfindung betrifft ein Verfahren zur Bestimmung der Drehwüchsigkeit eines Holzstammes.

Des Weiteren betrifft die Erfindung eine Verwendung einer Einrichtung zum Ermitteln von Höhenprofilen an der Oberfläche eines Gegenstandes.

Des Weiteren betrifft die Erfindung eine Sägewerksvorrichtung mit einer Einrichtung zur Bestimmung der Qualität eines Holzstammes.

Zurzeit werden Verfahren zur Klassifizierung der Qualität von Holzstämmen oder Teilen davon angewendet, die auf dem Vorhandensein von Ästen im Holzstamm beruhen. Dabei wird beispielsweise der Holzstamm durchleuchtet und auf Basis des unterschiedlichen Absorptionsverhaltens der Äste im Vergleich mit dem übrigen Stamm auf die Zahl der Äste im Holzstamm und damit eine Qualität des Holzes geschlossen. Die erhaltene Information wird dann weiterverarbeitet und das Holz, zumeist schon im geschnittenen Zustand, entsprechend sortiert. Mit einem solchen Verfahren lässt sich jedoch nicht auf eine Drehwüchsigkeit des Holzes schließen.

Die Drehwüchsigkeit eines Holzstammes bzw. die Abweichung eines gewachsenen Holzstammes von einem idealen Wachstum entlang bzw. parallel zur Längsachse des Stammes ist allerdings für die Praxis ein viel wichtigeres Qualitätskriterium. Wird die Drehwüchsigkeit eines Holzes nicht bestimmt, was bei aktuellen Sägewerksanlagen derzeit in keinem der automatischen Arbeitsschritte erfolgt, so kann dies zu großen finanziellen Einbußen führen. Insbesondere kann sich bereits geschnittenes, drehwüchsiges Holz im Lager bzw. beim Trocknen aufgrund der Drehwüchsigkeit verformen. Folge ist, dass das Holz, beispielsweise Kantholz, für beabsichtigte Verwendungszwecke, z. B. im Baugewerbe, unbrauchbar wird.

Aufgabe der Erfindung ist es, diesen Nachteil zu vermeiden und ein einfaches Verfahren zur Bestimmung der Drehwüchsigkeit von Holzstämmen anzugeben, sodass stark drehwüchsige Holzstämme aussortiert werden können.

Diese Aufgabe wird durch ein Verfahren zur Bestimmung der Drehwüchsigkeit eines Holzstammes gelöst, bei dem der Holzstamm zumindest teilweise entrindet wird und in einem entrindeten Bereich ein allenfalls von einem parallel zur Längsachse des Holzstammes abweichender Verlauf der Längsriefen des Holzstammes ermittelt und daraus die Drehwüchsigkeit bestimmt wird.

Die mit der Erfindung erzielten Vorteile sind insbesondere darin zu sehen, dass im praktischen Betrieb, beispielsweise in einem Sägewerk, auf einfache Weise und ohne erhebliche Zusatzkosten Information über die Drehwüchsigkeit eines Holzstammes erhalten werden kann. Dies erlaubt es in der Folge, die einzelnen Holzstämme, vor oder nach einem Schneiden, in Klassen verschiedener Holzqualität einzuteilen. Je nach Qualität des Holzes kann dieses dann seiner Güte angepasst eingesetzt werden. Insbesondere kann ein unnützes Lagern und Trocknen von drehwüchsigem Holz mit einhergehendem Verzug dadurch ausgeschlossen werden.

Neben der leichten Integrierbarkeit des erfindungsgemäßen Verfahrens in bestehende Prozesse besteht ein weiterer Vorteil darin, dass die Verdrehung eines Holzstammes bzw. die Drehwüchsigkeit von außen erkannt werden kann, also noch bevor der Holzstamm in einzelne Bretter geschnitten ist.

Der Holzstamm wird während der Bestimmung der Drehwüchsigkeit bevorzugt in Längsrichtung bewegt, um den Verlauf der Längsriefen über die gesamte Länge des Holzstammes zu erfassen. Dazu kann beispielsweise ein Förderband in einem Sägewerk dienen, über welchem eine entsprechende Erfassungseinrichtung stationär angeordnet ist.

Bevorzugt ist es, dass ein Verlauf der Längsriefen relativ zur Längsachse des Holzstammes optisch ermittelt wird, wenngleich auch andere Methoden, beispielsweise ein mechanisches Abtasten, angewendet werden können.

Insbesondere bei Einsatz optischer Methoden empfiehlt es sich, dass entlang der Längsachse des Holzstammes an mehreren Positionen Höhenprofile ermittelt werden und aus den Daten der Höhenprofile eine Drehwüchsigkeit des Holzstammes berechnet wird.

Die Höhenprofile können beispielsweise mittels eines bekannten Triangulationsverfahrens ermittelt werden.

Alternativ zur Triangulation ist es auch möglich, eine weitere optische Methode anzuwenden, wobei ein Bereich entlang der Längsachse des Holzstammes mit einer Lichtquelle beleuchtet und von diesem Bereich mit einer Kamera, deren optische Achse zur Beleuchtungsrichtung versetzt ist, ein Bild aufgenommen wird und anhand eines Verlaufs der im so aufgenommenen Bild erkennbaren Längsriefen eine Drehwüchsigkeit des Holzstammes berechnet wird.

Weitere Merkmale, Vorteile und Wirkungen der Erfindung ergeben sich aus den nachfolgenden Ausführungsbeispielen, anhand derer die Erfindung noch weitergehend erläutert ist. Es zeigen:

Fig. 1 einen Ausschnitt aus einem Holzstamm;
Fig. 2 Detailansichten von Längsriefen eines Holzstammes;
Fig. 3 eine schematische Darstellung einer Triangulationseinrichtung;
Fig. 4 eine schematische Darstellung einer Ermittlung der Höhenprofile auf einem Holzstamm mittels Triangulation;
Fig. 5 eine Darstellung der Höhenprofile eines Holzstammes, ermittelt durch Triangulation;
Fig. 6 die Höhenprofile aus Fig. 5 mit eingezeichnetem Verlauf einiger Längsriefen relativ zur Längsachse des Holzstammes;
Fig. 7 eine schematische Darstellung einer weiteren Einrichtung zur Bestimmung des Verlaufs von Längsriefen;

Fig. 8 eine Draufsicht auf die Längsriefen eines Holzstammes;

Fig. 9 die in Fig. 8 gezeigten Längsriefen nach gedachter Abwicklung der Oberfläche des Stammes und Ausbreitung in einer Ebene;

Fig. 10 das Sichtfeld der Kamera der in Fig. 7 gezeigten Einrichtung auf Längsriefen eines Holzstammes;

Fig. 11 eine mit einer Einrichtung gemäß Fig. 7 aufgenommene Fotografie der Oberfläche eines entrindeten Holzstammes;

Fig. 12 die in Fig. 11 gezeigte Fotografie mit eingezeichneten Längsriefen;

Fig. 13 eine schematische Wiedergabe der in Fig. 12 und Fig. 13 dargestellten Situation.

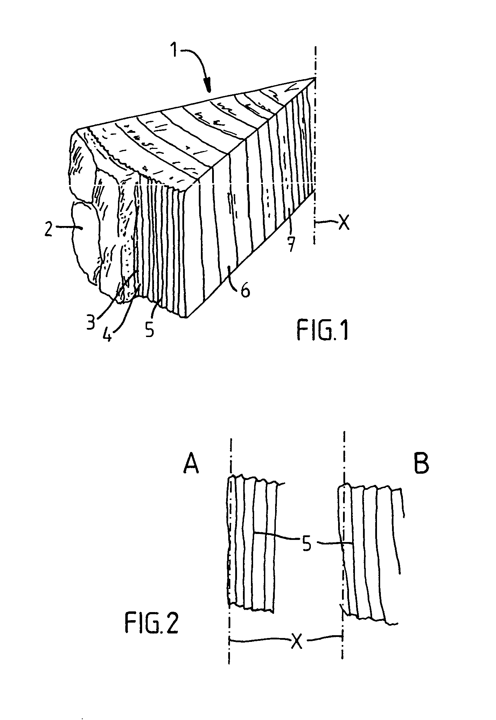

In Fig. 1 ist ein Ausschnitt eines Holzstammes 1 näher dargestellt. Der Holzstamm 1 besteht, von der Außenseite zum Inneren hin betrachtet, aus Borke 2, Bast 3, Kambium 4 sowie Splintholz 6 und Kernholz 7. In einem teilweise entrindeten Bereich des Holzstammes 1 sind Längsriefen 5 ersichtlich, die sich teilweise bis in das Kambium 4 bzw. den Bast 3 fortsetzen. Ist der Holzstamm 1 optimal gewachsen, so verlaufen die Längsriefen 5 exakt parallel zur Längsachse X des Holzstammes 1.

In Fig. 2 ist in der Detailansicht A ein optimaler Verlauf der Längsriefen 5 dargestellt, nämlich parallel zur Längsachse X des Holzstammes 1. Bei einem solchen Verlauf der Längsriefen 5 ist eine hohe Holzqualität gegeben. Ist hingegen ein Verlauf der Längsriefen 5 gegeben, wie er in der Detailansicht B der Fig. 2 skizziert ist, also kein paralleler Verlauf zur Längsachse X, so liegt eine Drehwüchsigkeit des Holzstammes 1 vor und weist das Holz eine schlechtere Qualität als ein solches gemäß Detailansicht A auf. Durch Ermittlung dieses vom optimalen, achsparallelen Verlauf abweichenden Längsriefenverlaufs kann eine Drehwüchsigkeit des Holzes bestimmt werden, was nachfolgend eine Klassifizierung des Holzes bzw. eine Einteilung in Qualitätsklassen erlaubt.

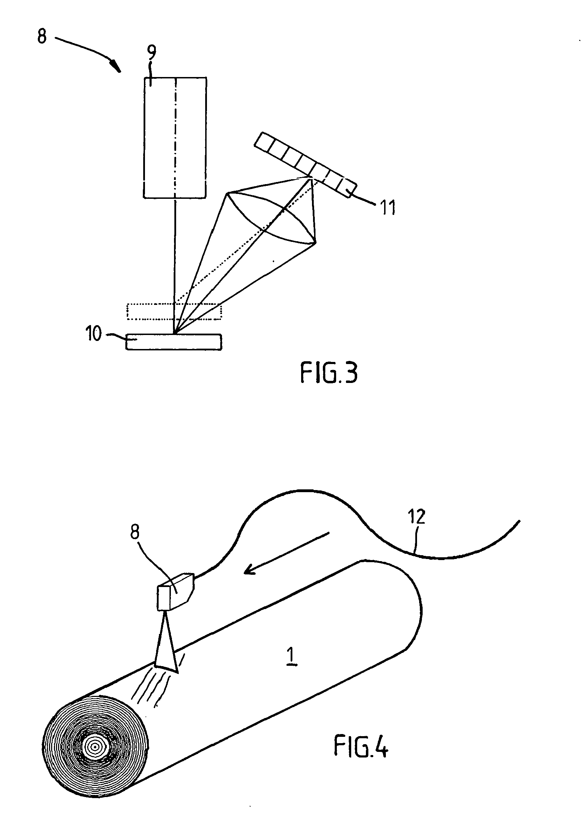

Für diese Bestimmung kann ein Triangulationsverfahren angewendet werden. Fig. 3 zeigt eine prinzipielle Anordnung zur Durchführung dieses Verfahrens. Dabei wird mit einem Laser 9 einer Triangulationseinrichtung 8 monochromatisches Licht auf die Oberfläche eines Messobjektes 10 gestrahlt und in einem vom Einstrahlungswinkel von 90° verschiedenen Winkel mittels eines CCD-Sensors 11 oder eines positionsempfindlichen Detektors das reflektierte Licht detektiert. Je nach punktueller Höhe des Messobjektes 10 wird bei der vorgesehenen Messgeometrie vom Messobjekt 10 reflektiertes Licht in unterschiedlichen Bereichen des CCD-Sensors 11 detektiert, woraus ein Höhenprofil des Holzstammes 1 quer zu dessen Längsachse X festgestellt bzw. berechnet werden kann. Bei diesem Verfahren kann das vom Laser 9 emittierte Licht moduliert werden, um Einflüsse von Fremdlicht für die Ermittlung der einzelnen Höhenprofile zu vermindern.

Im praktischen Einsatz kann die Triangulationseinrichtung 8, wie in Fig. 4 gezeigt, aus einer stationären Einheit bestehen, unter welcher der Holzstamm 1 vorbeigeführt wird, beispielsweise mit einem Förderband. In diesem Fall, also bei Bewegung des Holzstammes 1, ist es wichtig, dass eine Abtastrate des Sensors 11 bzw. der Triangulationseinrichtung 8 wie auch eine Transportgeschwindigkeit des Holzstammes 1 konstant ist, damit aus den ermittelten Höhenprofilen auch zuverlässig bzw. ohne Fehler auf den Versatz einzelner Längsriefen 5 bzw. eine Drehwüchsigkeit des Holzstammes 1 geschlossen werden kann. Zweckmäßig ist es weiter, dass eine relativ große Abtastrate zwischen einzelnen Profillinien vorgesehen wird, damit der Verlauf einzelner Längsriefen 5 in aufeinanderfolgenden Höhenprofilen verfolgt werden kann. Insbesondere sollte ein Abstand zwischen zwei Abtastungen bzw. Scans, am Holzstamm 1 gemessen, nicht mehr als 2 Millimeter betragen. Hierfür wird die Transportgeschwindigkeit des Holzstammes 1 in der in Fig. 4 durch einen Pfeil angedeuteten Richtung auf einen geeigneten Wert eingestellt.

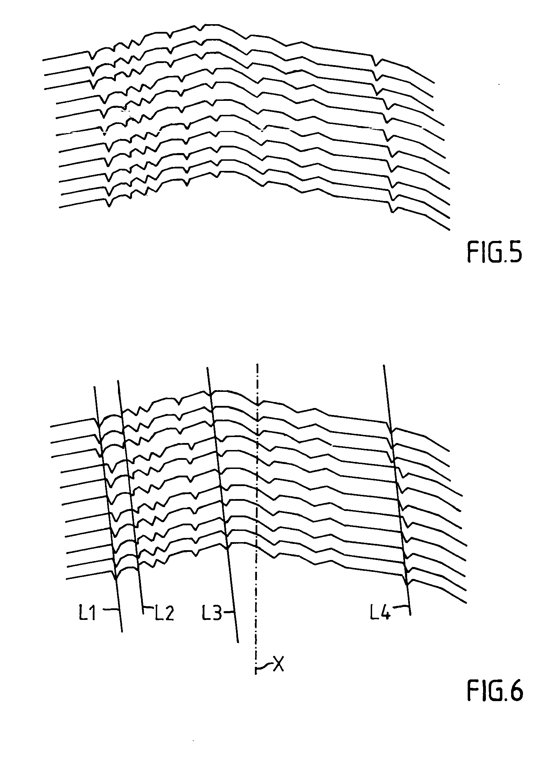

Die mit der Triangulationseinrichtung 8 aufgenommenen Daten werden mittels eines Kabels 12 einer Auswerteeinheit wie einem Computer übermittelt, mit welchem vorzugsweise vollautomatisch die weitere Datenverarbeitung bzw. Bestimmung der Drehwüchsigkeit des Holzstammes 1 durchgeführt wird. Dazu wird der Verlauf einzelner Zacken in den nacheinander aufgenommenen Höhenprofilen, wie sie anhand eines Beispieles in Fig. 5 dargestellt sind, verfolgt. In mehreren aufeinanderfolgenden Höhenprofilen aufscheinende Zacken korrespondieren zu einer einzelnen Längsriefe 5 am Holzstamm 1. Durch Bestimmung des Winkels einer Geraden entlang korrespondierender Zacken relativ zur Längsachse X des Holzstammes 1 kann direkt auf eine Winkelneigung der Längsriefen 5 bzw. eine Verdrehung des Holzstammes 1 geschlossen werden. Diese Situation ist in Fig. 6 dargestellt, worin die einzelnen Linien L1, L2, L3, L4 jeweils dem Verlauf einer Längsriefe 5 entsprechen. So kann beispielsweise bei den in Fig. 6 dargestellten Höhenprofilen ein Winkel der Längsriefen 5 zur Längsachse X des Holzstammes 1 von etwa 7° ermittelt werden. Damit diese Bestimmung möglichst exakt erfolgen kann, ist es wie bereits erwähnt erforderlich, dass sowohl die Abtastrate der Triangulationseinrichtung 8 als auch eine Transportgeschwindigkeit des Holzstammes 1 möglichst konstant ist. Dies kann erreicht werden, wenn die Triangulationseinrichtung 8 mit einer fixen Abtastrate arbeitet und eine Transportgeschwindigkeit des Holzstammes 1 durch Geschwindigkeitsmessung, beispielsweise mithilfe eines Impulsgebers oder mit einem optischen Geschwindigkeitsmesssystem, erfasst, kontrolliert und gegebenenfalls geregelt wird.

Für die Auswertung, insbesondere die automatisierte Auswertung mit einem Computer oder Mikrocontroller, kann die momentane Transportgeschwindigkeit des Holzstammes 1 der Auswerteeinheit übermittelt werden, sodass der Abstand zwischen den einzelnen Höhenprofilen berechnet werden kann. Danach wird anhand von markierten Zacken bzw. Rillen dieselbe Rille in einem darauffolgenden Höhenprofil gesucht und deren Position festgestellt. Für die Identifizierung und Zuordnung einer einzelnen Rille zu der entsprechenden Rille im vorausgegangenen bzw. nachfolgenden Höhenprofil werden verschiedene Merkmale, beispielsweise Rillentiefe, Rillenbreite, Position der Rille oder Ähnliches herangezogen. Aus den so bekannten Positionsänderungen einzelner Rillen relativ zu ihrer vorangegangenen Position kann nun über den Abstand zwischen den zwei Höhenprofilen ein Winkel ermittelt werden. Dabei gilt

$$\tan \alpha = (P1.1 - P1)/L,$$

wobei P1 der Position im vorangegangenen Höhenprofil entspricht, P1.1 der Position im aktuellen Höhenprofil entspricht, L den Abstand zwischen zwei Höhenprofilen bedeutet und α den Winkel in Grad darstellt. Es werden alle Winkel der erfassten Rillen der Höhenprofile ermittelt. Aus dieser Reihe von Winkelwerten wird danach der Medianwert ermittelt. Schließlich wird mit Hilfe dieser Berechnung die Verdrehung bzw. Drehwüchsigkeit des Holzstammes 1 zwischen den beiden Höhenprofilen ermittelt. Dieses Verfahren bzw. Berechnungsmethode wird bei allen Höhenprofilen des Holzstammes 1 angewendet. Dadurch ergibt sich schließlich die gesamte Verdrehung des Holzstammes 1 in zuverlässiger Weise.

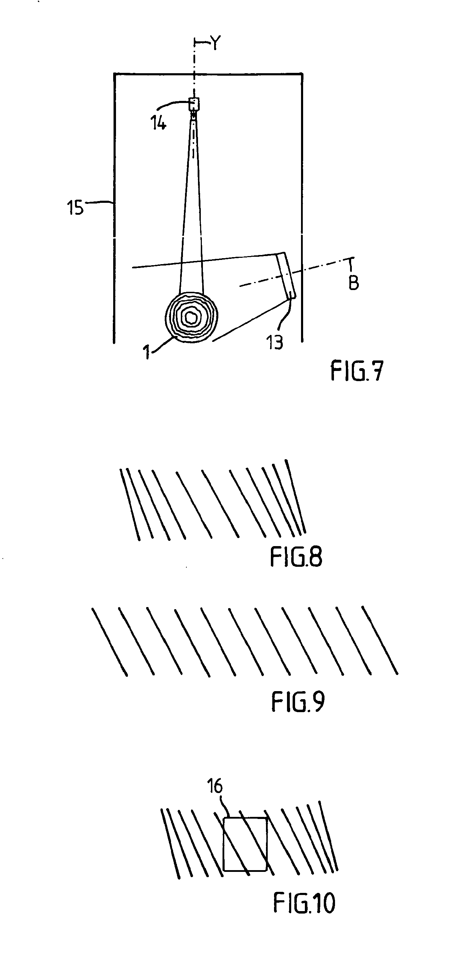

Neben der Triangulation können auch andere optische Verfahren bzw. Einrichtungen zur Bestimmung der Drehwüchsigkeit eines Holzstammes 1 eingesetzt werden. Beispielsweise ist es möglich, mit einer Einrichtung gemäß Fig. 7 den Verlauf von Längsriefen 5 eines zumindest teilweise entrindeten Holzstammes 1 relativ zu dessen Längsachse X festzustellen. Die Vorrichtung umfasst dabei ein Gehäuse 15, in welchem eine Kamera 14 und eine Beleuchtungseinheit 13 angeordnet sind. Ähnlich wie bei dem beschriebenen Triangulationsverfahren kann der Holzstamm 1 während der Messung in Richtung seiner Längsachse X transportiert werden und wird dabei von der Beleuchtungseinheit 13 in Richtung einer Beleuchtungsrichtung B beleuchtet. Über dem Holzstamm 1 ist eine Kamera 14 angeordnet, deren optische Achse Y von der Beleuchtungsrichtung B abweicht. Dies ist notwendig, um eine Erkennung der feinen Längsriefen 5 des Holzstammes 1 zu ermöglichen, denn dadurch erscheinen die Längsriefen 5 im Bild der Kamera mit einem Schatten. Ein Winkel zwischen der Beleuchtungsrichtung B und der optischen Achse Y der Kamera 14 kann zweckmäßigerweise in einem Bereich von 15° bis 65° liegen. Die Helligkeitsunterschiede werden von der Kamera oberhalb der beleuchteten Stammfläche erfasst. Die Erfassung der Helligkeitsunterschiede erfolgt, wie bei optoelektronischen Kamerasystemen üblich, durch lichtempfindliche Sensoren, bei denen der Helligkeitswert in einen Spannungswert umgewandelt wird und mehrere Spannungswerte zu einem Array zusammengeschlossen werden. Die einzelnen Pixel werden zu einem Bild zusammengestellt und an eine Auswerteeinheit, beispielsweise einen Mikrocontroller oder Computer, weitergeleitet.

Die Beleuchtung ist so ausgelegt, dass sie eine etwa quadratische Fläche bildet, deren Seiten- und Höhenverhältnisse am Holzstamm 1 einerseits von der Beobachtungsgröße der Kamera bzw. deren Aufnahmefeld und andererseits von einem minimalen und maximalen Baumstammdurchmesser bestimmt werden. Der Beleuchtungskörper sollte so gewählt werden, dass er die Gesamtfläche des Holzstammes 1 gleichmäßig ausleuchtet und den Gesamthöhenbereich zwischen dem kleinsten zu prüfenden Holzstamm und dem größten zu prüfenden Holzstamm abdecken kann. Die Beleuchtungseinrichtung 13 sollte auch einen geringen Öffnungswinkel für die emittierte Strahlung haben, damit die notwendige Schattenbildung am Holzstamm 1 nicht durch schräg von oben einfallende Strahlung aufgehoben wird. Im Zusammenhang damit dient auch das Gehäuse 15 dazu, einen Einfluss von Fremdstrahlung auszuschalten.

Bei der Kamerapositionierung ist es wichtig, diese mit Bezug auf den Holzstamm 1 so zentral wie möglich über der Stammmitte bzw. Längsachse X zu wählen, damit die Ausrichtung der Längsriefen 5 möglichst fehlerfrei erfasst werden kann. Die erfasste Ausrichtung der Längsriefen 5 tendiert nämlich mit zunehmender Abweichung von der Stammmitte bzw. Längsachse X immer mehr gegen 0°. Dieser Effekt ist durch die Krümmung des Holzstammes 1 bedingt, da die Kamera 14 nur einen limitierten Bereich der Oberfläche des Holzstammes 1 aufnimmt und dadurch Längen- und Höhenverhältnisse am Rand des Holzstammes 1 nicht korrekt wiedergeben kann. Dieser Effekt wird durch Vergleich der Fig. 8 und der Fig. 9 deutlich, wobei Fig. 8 eine Betrachtung auf einen gekrümmten Holzstamm 1 aus dem Blickwinkel der Kamera 14 zeigt, wohingegen Fig. 9 dieselbe Oberfläche, jedoch abgewickelt in eine Ebene, zeigt. Um diesem Effekt entgegenzutreten, werden zwei Strategien angewendet. Zum einen wird nur ein eingeschränktes Sichtfeld 16 des Holzstammes 1 aufgenommen, da dann dieser Effekt noch keine Fehlmessungen verursacht, und andererseits wird die Krümmung des Holzstammes 1 im Messfeld bestimmt und die entsprechenden Längsriefen 5 mit ihrer Ausrichtung anhand ihrer Position zur Stammmitte bzw. Längsachse X korrigiert. Das Sichtfeld 16 der Kamera 14 beträgt mit Bezug auf eine Breite des Holzstammes 1, wie in Fig. 10 dargestellt, bevorzugt weniger als 25 %.

Die Ermittlung der Kameraposition relativ zur Stammmitte bzw. Längsachse X erfolgt durch Erfassung einer Seitenkante des Holzstammes 1, wodurch die Krümmung der Stammoberfläche bei der Erfassung der Ausrichtung berücksichtigt werden kann. Diese Korrekturberechnung wird mit folgender Formel definiert:

$$\alpha 1 = \arctan (\tan \alpha / \cos \beta),$$

wobei $\alpha$ den Winkel der ermittelten Längsrille 5, $\beta$ den Winkel der Tangente im Schwerpunkt der Längsriefe 5 am Stammradius und $\alpha 1$ den korrigierten Winkel der Längsriefe 5 bedeuten. Diese Korrektur ist zwar eine idealisierte Annahme des Verlaufs der Krümmung des Holzstammes 1, bietet jedoch eine ausreichende Genauigkeit für die Korrektur der Ausrichtung einer Längsriefe 5 abhängig von der Position derselben auf der Baumstammgeometrie.

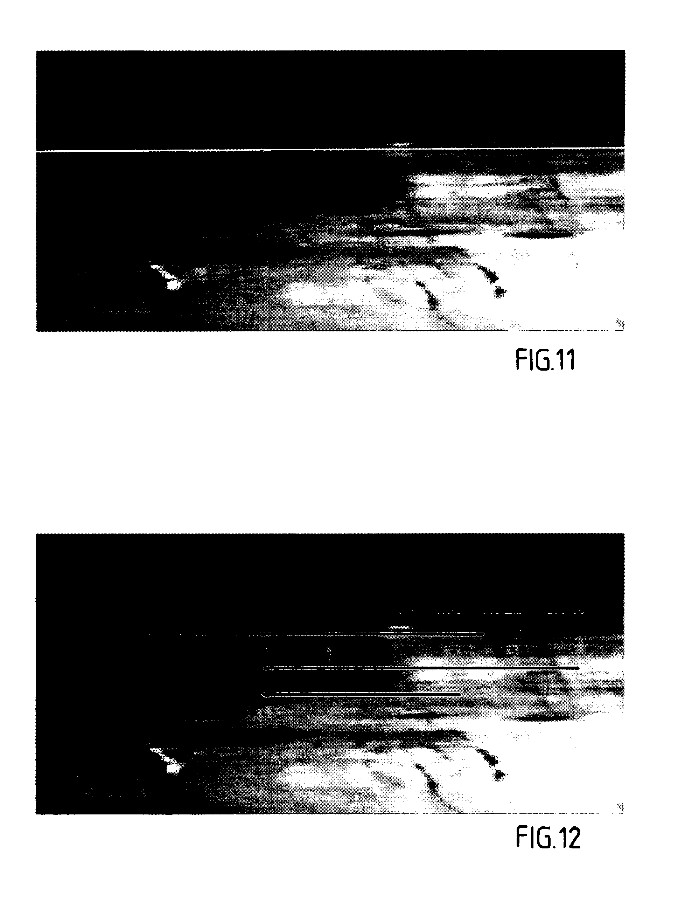

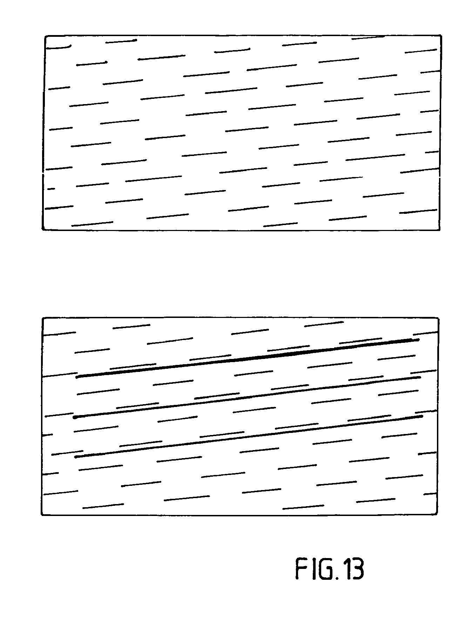

Anhand der aufgenommenen Kamerabilder können mittels Algorithmen zur Bildverarbeitung die einzelnen Längsriefen 5 detektiert und deren Ausrichtung bestimmt werden. Die gefundenen Längsriefen 5 werden anschließend noch anhand des vorstehend erläuterten Verfahrens korrigiert, wobei, um einen repräsentativen Winkel für diesen Aufnahmebereich zu ermitteln, von allen aufgefundenen Längsriefen 5 der Medianwert errechnet wird. Fig. 11 zeigt eine typische Aufnahme, anhand derer Längsriefen 5 ermittelt werden können, welche in Fig. 12 durch Striche angedeutet sind. Fig. 13 zeigt eine schematische Darstellung einer realen Situation, wie sie beispielsweise in Fig. 11 und Fig. 12 gezeigt ist.

Wenn eine der beiden vorstehend dargestellten Einrichtungen zur Bestimmung der Drehwüchsigkeit eines Holzstammes in einem Sägewerk eingesetzt wird, beispielsweise in einem Bereich der für die Sortierung von Holz in verschiedene Qualitäten vorgesehen ist, soll die Einrichtung selbstverständlich nur dann messen und auswerten, wenn ein Holzstamm bearbeitet bzw. sortiert wird. Zu diesem Zweck können die Messeinrichtungen mit einer Lichtschranke oder einem dergleichen System kombiniert sein, welches auf die Anwesenheit eines Holzstammes 1 schließen lässt und die Messeinrichtung aktiviert.

Die vorstehend beschriebenen Einrichtungen und Auswerteeinheiten zur Bestimmung der Drehwüchsigkeit von Holzstämmen werden bevorzugt in Sägewerkseinrichtungen eingesetzt, und zwar entlang von Arbeitsstraßen, auf welchen die Holzstämme transportiert werden. Eine Bestimmung der Drehwüchsigkeit von Holzstämmen kann in diesem Fall ohne Veränderung der üblichen Prozesse in einem Sägewerk erfolgen.

Verfahren und Vorrichtungen zum Entrinden von Holzstämmen, welche eine Oberfläche der Holzstämme soweit unversehrt lassen, dass eine Drehwüchsigkeit auf erfindungsgemäße Weise bestimmt werden kann, zählen zum Stand der Technik.

Das erfindungsgemäße Verfahren eignet sich insbesondere für Nadelhölzer mit ausgeprägter Struktur der Längsriefen, kann aber auch für Laubhölzer nutzbringend eingesetzt werden.

**Patentansprüche**

1. Verfahren zur Bestimmung der Drehwüchsigkeit eines Holzstammes (1), **dadurch gekennzeichnet, dass** der Holzstamm (1) zumindest teilweise entrindet wird und in einem entrindeten Bereich ein allenfalls von einem parallel zur Längsachse (X) des Holzstammes (1) abweichender Verlauf der Längsriefen (5) des Holzstammes (1) ermittelt und daraus die Drehwüchsigkeit bestimmt wird.

2. Verfahren nach Anspruch 1, **dadurch gekennzeichnet, dass** ein Verlauf der Längsriefen (5) relativ zur Längsachse (X) des Holzstammes (1) optisch ermittelt wird.

3. Verfahren nach Anspruch 1 oder 2, **dadurch gekennzeichnet, dass** entlang der Längsachse (X) des Holzstammes (1) an mehreren Positionen Höhenprofile ermittelt werden und aus den Daten der Höhenprofile eine Drehwüchsigkeit des Holzstammes (1) berechnet wird.

4. Verfahren nach Anspruch 3, **dadurch gekennzeichnet, dass** die Höhenprofile mittels Triangulation ermittelt werden.

5. Verfahren nach Anspruch 1 oder 2, **dadurch gekennzeichnet, dass** ein Bereich entlang der Längsachse (X) des Holzstammes (1) mit einer Lichtquelle (13) beleuchtet und von diesem Bereich mit einer Kamera (14), deren optische Achse (Y) zur Beleuchtungsrichtung (B) versetzt ist, ein Bild aufgenommen wird und anhand eines Verlaufs der im so aufgenommenen Bild erkennbaren Längsriefen (5) eine Drehwüchsigkeit des Holzstammes (1) berechnet wird.

6. Verfahren nach einem der Ansprüche 2 bis 5, **dadurch gekennzeichnet, dass** der Holzstamm (1) bei der Bestimmung der Drehwüchsigkeit beispielsweise mit einem Förderband in Richtung seiner Längsachse (X) bewegt wird.

7. Verwendung einer Einrichtung zum Ermitteln von Höhenprofilen an der Oberfläche eines Gegenstandes zur Bestimmung des Verlaufs von Längsriefen (5) eines Holzstammes (1) relativ zu dessen Längsachse (X).

8. Sägewerksvorrichtung mit einer Einrichtung zur Bestimmung der Qualität eines Holzstammes (1), **dadurch gekennzeichnet, dass** die Einrichtung eine optische Einrichtung zur Aufnahme eines Verlaufs von Längsriefen (5) eines zumindest teilweise entrindeten Holzstammes (1) und zumindest ein Transportmittel, mit dem die optische Einrichtung relativ zum Holzstamm (1) entlang dessen Längsachse (X) oder umgekehrt bewegbar ist, beispielsweise ein

**EP 1 895 298 A1**

Förderband, sowie eine Auswerteeinrichtung umfasst.

FIG.1

A        B

FIG.2

X

FIG.3

FIG.4

FIG.5

FIG.6

FIG.7

FIG.8

FIG.9

FIG.10

FIG.11

FIG.12

FIG.13

**Europäisches Patentamt**

# EUROPÄISCHER RECHERCHENBERICHT

Nummer der Anmeldung

EP 07 45 0147

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2003/079544 A1 (FLOYD STANLEY L [US]) 1. Mai 2003 (2003-05-01) * Absätze [0001], [0003], [0008], [0009] * * Absätze [0013] - [0019], [0037], [0039], [0041] - [0044], [0047] - [0049] * * Absätze [0061] - [0090] * * Abbildungen 4-21 * ----- | 1 | INV. G01N33/46 G01N21/898 |
| X | WO 03/104777 A (IND RES LTD [NZ]; ANDREWS MICHAEL KENNETH [NZ]) 18. Dezember 2003 (2003-12-18) * Seite 20, Zeile 24 - Seite 21, Zeile 9 * * Seite 25, Zeile 1 - Seite 27, Zeile 8 * * Abbildungen 1-15 * ----- | 1-8 | |
| X | US 6 308 571 B1 (STANISH MARK A [US] ET AL) 30. Oktober 2001 (2001-10-30) * Spalte 1, Zeile 25 - Spalte 3, Zeile 39 * * Spalte 9, Zeile 20 - Zeile 27 * * Spalte 10, Zeile 35 - Zeile 46 * * Spalte 11, Zeile 11 - Zeile 18; Abbildungen 2-5; Beispiel 3 * * Ansprüche 56-66 * ----- | 1-8 | RECHERCHIERTE SACHGEBIETE (IPC) G01N G01B |
| X | R. WINN HARDIN: "Automation speeds sawmill inspection" VISION SYSTEM DESIGN, [Online] April 2006 (2006-04), Seiten 43-48, XP002461579 Gefunden im Internet: URL:http://www.vsd-digital.com/vsd/200604/?pg=45> [gefunden am 2007-12-06] * Seiten 43,46 * ----- | 7,8 | |

-/--

Der vorliegende Recherchenbericht wurde für alle Patentansprüche erstellt

| Recherchenort | Abschlußdatum der Recherche | Prüfer |
|---|---|---|
| München | 10. Dezember 2007 | Consalvo, Daniela |

**EP 1 895 298 A1**

| Europäisches Patentamt | **EUROPÄISCHER RECHERCHENBERICHT** | **Nummer der Anmeldung**<br>EP 07 45 0147 |
|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 4 606 645 A (MATTHEWS PETER C [GB] ET AL) 19. August 1986 (1986-08-19)<br>* Spalte 1, Zeile 7 - Zeile 21; Abbildungen 12-19 *<br>* Spalte 9, Zeile 47 - Spalte 11, Zeile 29 * | 1-8 | |
| A | EP 1 630 519 A (MICROTEC S R L [IT]) 1. März 2006 (2006-03-01)<br>* Absätze [0020] - [0027], [0055]; Abbildung 3 * | 1-8 | |
| A | US 2002/024677 A1 (METCALFE LEONARD [CA] ET AL) 28. Februar 2002 (2002-02-28)<br>* Absätze [0008] - [0019]; Abbildungen 1-5 * | 1-8 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 10. Dezember 2007 | Consalvo, Daniela |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**          EP 07 45 0147

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-12-2007

| Im Recherchenbericht angeführtes Patentdokument | | Datum der Veröffentlichung | Mitglied(er) der Patentfamilie | | Datum der Veröffentlichung |
|---|---|---|---|---|---|
| US 2003079544 | A1 | 01-05-2003 | CA | 2406851 A1 | 30-04-2003 |
| | | | NZ | 521670 A | 24-09-2004 |
| WO 03104777 | A | 18-12-2003 | AU | 2003238741 A1 | 22-12-2003 |
| | | | AU | 2003238742 A1 | 22-12-2003 |
| | | | WO | 03104776 A1 | 18-12-2003 |
| | | | NZ | 519475 A | 29-10-2004 |
| US 6308571 | B1 | 30-10-2001 | KEINE | | |
| US 4606645 | A | 19-08-1986 | CA | 1239476 A1 | 19-07-1988 |
| | | | DE | 3582161 D1 | 18-04-1991 |
| | | | EP | 0198037 A1 | 22-10-1986 |
| | | | FI | 862741 A | 26-06-1986 |
| | | | JP | 62500684 T | 19-03-1987 |
| | | | WO | 8602729 A1 | 09-05-1986 |
| EP 1630519 | A | 01-03-2006 | KEINE | | |
| US 2002024677 | A1 | 28-02-2002 | KEINE | | |

EPO FORM P0461

Für nähere Einzelheiten zu diesem Anhang : siehe Amtsblatt des Europäischen Patentamts, Nr.12/82